Europäisches Patentamt

European Patent Office

Office européen des brevets

(19)

(11) Publication number: **0 176 349**
**A2**

(12) # EUROPEAN PATENT APPLICATION

(21) Application number: **85306789.0**

(22) Date of filing: **24.09.85**

(51) Int. Cl.⁴: **C 12 N 15/00**
C 12 N 5/00, C 12 N 9/00
C 12 N 9/99, C 12 P 13/22
C 12 P 13/08, C 12 P 13/12
//C12R1:19

(30) Priority: **24.09.84 US 653193**
**18.03.85 US 713297**

(43) Date of publication of application:
**02.04.86 Bulletin 86/14**

(84) Designated Contracting States:
**AT BE CH DE FR GB IT LI NL SE**

(71) Applicant: **BIOTECHNICA INTERNATIONAL, INC.**
**85 Bolton Street**
**Cambridge Massachusetts(US)**

(72) Inventor: **Backman, Keith C.**
**200 Carlisle Road**
**Bedford Massachusetts(US)**

(74) Representative: **Deans, Michael John Percy et al,**
**Lloyd Wise, Tregear & CO. Norman House 105-109**
**Strand**
**London WC2R OAE(GB)**

(54) Methods of altering cells to increase production of a desired compound, cells capable of biosynthesis of a desired compound, methods of producing desired compounds and methods of selecting mutant cells exhibiting increased production of a desired compound.

(57) Cells are altered to enhance production of a desired compound by circumventing feedback inhibition of an isozyme that catalyzes a reaction in the the biosynthetic pathway of a desired compound by relying on expression of a gene coding for a non-feedback inhibited isozyme. Also disclosed is a method for selecting mutant cells that have a feedback resistant enzyme that catalyzes a chemical reaction in the biosynthesis of both the desired compound and in the biosynthes of a second compound that is essential for cell growth. The selection is accomplished by growing the cells in feedback-inhibiting concentrations of the inhibitor (i.e., the desired compound or one of its metabolic precursors) and under conditions in which catalytic activity of the enzyme is required for cell growth. Mutants capable of cell growth have avoided enzyme inhibition.

./...

FIG. I

## METHODS OF ALTERING CELLS TO INCREASE PRODUCTION OF A DESIRED COMPOUND, CELLS CAPABLE OF BIOSYNTHESIS OF A DESIRED COMPOUND, METHODS OF PRODUCING DESIRED COMPOUNDS AND METHODS OF SELECTING MUTANT CELLS EXHIBITING INCREASED PRODUCTION OF A DESIRED COMPOUND

This application relates in its various aspects to methods of altering cells to increase production of a desired compound, cells capable of biosynthesis of a desired compound, methods of producing desired compounds and methods of selecting mutant cells exhibiting increased production of a desired compound

A fundamental limitation of _in vivo_ methods of producing compounds is that organisms often have regulatory mechanisms to limit synthesis to amounts of the compound sufficient for the organism's own needs, thus avoiding waste of the organism's raw materials and energy. Regulation of biosynthesis may be effected by feedback inhibition of enzymes in the biosynthetic pathway, whereby the catalytic activity of the enzyme is substantially reduced when the concentration of an inhibiting compound reaches a certain level, the inhibiting compound being the desired compound or a metabolic precursor of it.

Various methods of avoiding feedback inhibition are known.

Synenki et al. EP 077196 discloses isolation of a gene specifying a DAHP synthase that is resistant to feedback inhibition by aromatic amino acids. The gene is isolated from a mutant E. coli strain that produces DAHP synthase in the presence of relatively high concentrations of aromatic amino acids.

It is known to isolate mutant organisms that are resistant to analogs of the desired product which are normally toxic because they inhibit enzymes used for product biosynthesis much as the product itself causes feedback inhibition. Organisms that are not killed by the analog may be free from feedback inhibition by the desired product as well as the analog. See, e.g. Tsuchida U.S. Patent 4,407,952 disclosing an E. coli mutant that is resistant to feedback inhibition by phenylalanine and a phenylalanine analog.

Regulation of biosynthesis also may be effected at the level of gene expression. For example, sufficiency or surfeit of a particular compound prevents expression of genes encoding one or more of the enzymes that catalyze reactions in the biosynthetic pathway of that compound. Genetic sequences, located in the vicinity of the structural gene coding for the enzyme, regulate expression of the enzyme. In some cases, more than one kind of regulatory sequence may be associated with a gene. Examples of such regulatory sequences include: promoters, operators, attenuators, antiterminators, ribosome binding sites, and sites for positive effectors.

Various ways of circumventing feedback-control of gene transcription are known and are referred to in the specification of our European Patent Application No 84306841.2 (Publication No 0145156), the disclosure of which is to be regarded as incorporated herein.

We describe below the alteration of a cell to increase its production of a desired compound by circumventing feedback inhibition of an enzyme in the biosynthetic pathway of that compound, the enzyme being a first isozyme that is feedback inhibited with respect to the desired compound. As used in this application, the term "feedback inhibited" with respect to a desired compound means that the enzyme's catalytic activity is substantially inhibited once the concentration of an inhibitor reaches a certain level, the inhibitor being the desired compound or a metabolic precursor on the compound's biosynthetic pathway. The term does not include enzyme inhibition by compounds that are on branches off that pathway leading to synthesis of compounds other than the desired compound. To circumvent feedback inhibition of the first isozyme, a compound-producing cell is transformed with a gene coding for a second isozyme that is substantially uninhibited at that inhibitor concentration level. The second isozyme gene is not subject to transcription regulation in response to such inhibitor concentrations, and so the second isozyme is produced at sufficient levels to enhance production of the desired compound.

Thus, according to a first aspect of this invention, we provide a method of altering a cell to increase its production of a desired compound via a biosynthetic pathway by circumventing feedback inhibition of a first isozyme, said first isozyme catalyzing a reaction in said pathway, the catalytic activity of said first isozyme being substantially inhibited at concentrations of an inhibitor exceeding a first concentration level, said inhibitor being said desired compound or a metabolic precursor to it; said method comprising: transforming said cell with DNA comprising a gene coding for a second isozyme, the catalytic activity of said second isozyme being substantially uninhibited at said first concentration level of said inhibitor, and said gene being transcribed at said inhibitor concentrations in amounts sufficient to enhance said product production.

In preferred embodiments, the cell is a member of the species E. coli, and the desired compound is the inhibitor; when the desired compound is an aromatic amino acid such as phenylalanine, the isozymes are coded by aro genes, e.g. the first isozyme is coded by aroG and the second isozyme is coded by aroF. Alternatively, the desired compound is threonine, methionine, or lysine, and the isozymes catalyze a step common to the biosynthesis of at least two of those compounds. Transcription of the second isozyme in the presence of the desired compound is enabled by providing sufficient copies of the gene in the cell to outnumber a transcription regulating effector. Alternatively, methods of evading transcription control described in our co-pending European Application 84306841.2 are used.

In a second and alternative aspect thereof, the invention provides a cell capable of biosynthesis of a desired compound, said biosynthesis comprising a reaction catalyzed by an isozyme that is resistant to feedback inhibition by said desired compound or its metabolic precursors, said isozyme being produced in said cell by a gene whose transcription is not feedback repressed by said desired compound or its metabolic precursors, said cell having been formed by transforming a precursor cell with DNA comprising said gene.

We also describe below the selection of a mutant cell that exhibits increased production of a desired compound over that of its parent cell population. The parent cells have a gene coding for a feedback inhibited enzyme catalyzing a chemical reaction common to the biosynthesis of both the desired compound and a second compound that is essential to cell growth. The parent cells are cultured in the presence of enzyme-inhibiting concentrations of that inhibitor, and mutants are selected on their ability to grow under conditions such that a growth-sustaining level of the second compound is available only as a result of the catalytic activity of the enzyme. Such mutants exhibit freedom from feedback inhibition.

Thus, the invention provides, in a third alternative aspect thereof, a method of selecting a mutant cell that exhibits increased production of a desired compound over production levels of a parent cell from which said mutant is formed, said parent cells comprising a gene coding for a feedback inhibited enzyme that catalyzes a chemical reaction common to the biosynthesis of said desired compound and of a second compound, said second compound being essential for cell growth; said method comprising: culturing said parent cell in the presence of an enzyme-inhibiting concentration of said inhibitor; and selecting mutants capable of growth under conditions such that a growth-sustaining level of said second compound is available as a result of the catalytic activity of said enzyme.

In preferred embodiments, the inhibitor is the desired compound, the enzyme is an isozyme, and the parent cell lacks the ability to produce other isozymes which catalyze the reaction effectively enough to provide growth-sustaining levels of the second compound under the selection conditions. The parent cell is preferably a cell formed by the aforesaid method of altering a cell to increase production of a desired compound via a biosynthetic pathway, the compound being an aromatic amino acid or one of the non-aromatic amino acids, threonine, methionine, or lysine. Where the enzyme is a second isozyme and it is inhibited not only by the second compound, but also to some extent (although only at much higher inhibitor concentrations) by the same inhibitor that controls the first isozyme, the method may be used to develop a gene for an isozyme in the desired compound's biosynthetic pathway that is not feedback inhibited by the desired compound, by the second compound, or by their metabolic precursors.

In preferred embodiments, the desired compound is produced by culturing a cell produced as described above in a liquid medium and recovering the desired compound from it.

It.will be seen that we take advantage of the existence of sets of enzymes (isozymes) that catalyze the same chemical reaction but are structurally and functionally distinct in their responses to feedback inhibition by the desired product or its metabolic precursors. The resulting organism is enabled to perform the reaction at issue using an isozyme that is not feedback inhibited in place of the naturally occurring feedback-inhibited isozyme. Compound production will be enhanced so long as sufficient copies of the deregulated isozyme are produced, a condition that is achieved by circumventing transcription regulation as described above. In this way the engineered cells avoid normal regulatory mechanisms and therefore do not curtail synthesis in response to elevated levels of the product. In many applications, as levels of the product increase, the product is released to the surrounding medium, thus making recovery of the desired compound more convenient.

Other features and advantages of the invention will be apparent from the following description of the preferred embodiments and from the claims.

We first briefly describe the drawings.

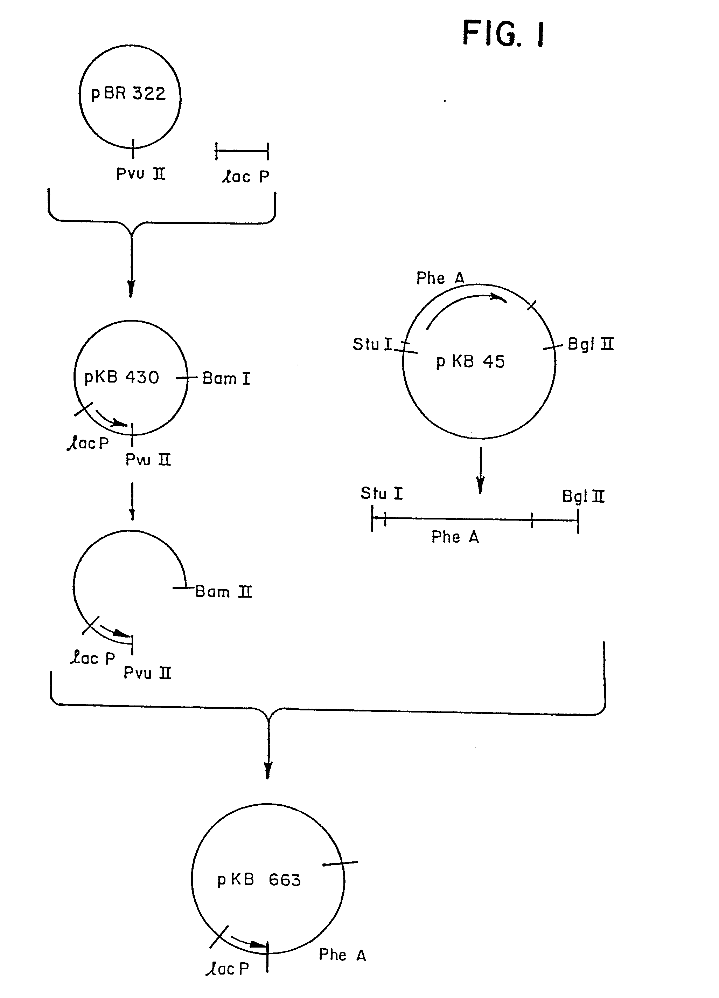

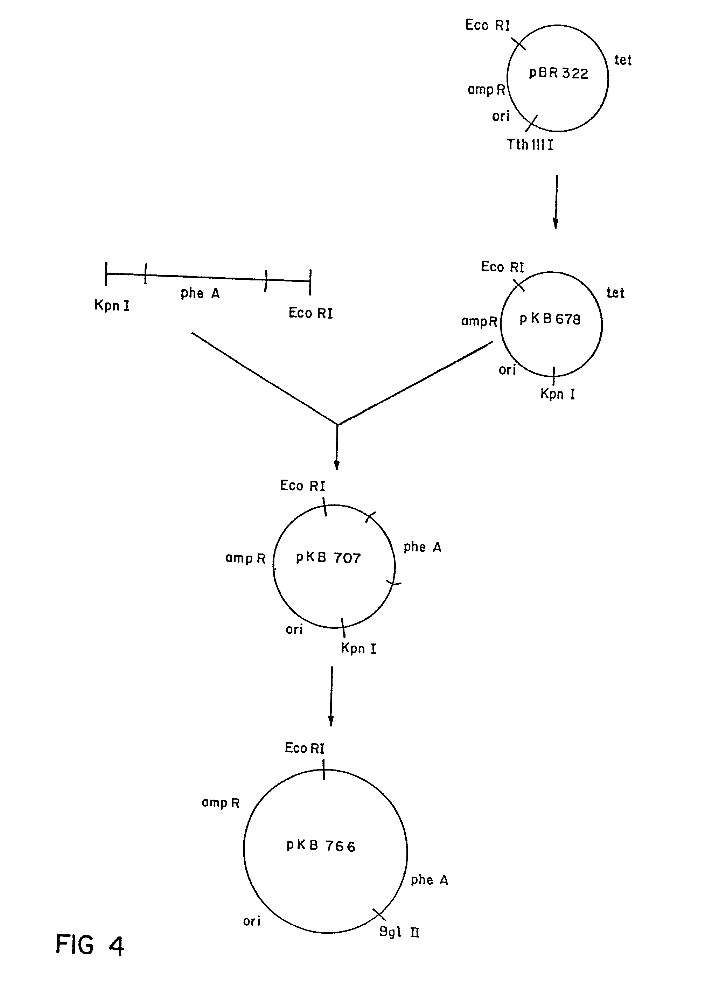

Figs. 1-4 are diagrams representing respectively steps in the engineering of plasmids pKB663, pKB712, pKB750, and pKB766.

Expression Vectors.

The desired product compound is synthesized via a synthesis pathway including at least one step that is ordinarily catalyzed by a feedback inhibited isozyme, so that, when the concentration of an inhibitor, usually the desired compound, reaches a certain level, the isozyme's catalytic action is prevented or severely reduced.

Such feedback inhibition is circumvented by enabling the cell to perform that reaction step with a different isozyme that is not feedback inhibited by the desired compound or its metabolic precursors. Even though the non-feedback inhibited isozyme is present in the cell naturally, its availability generally is insufficient for various reasons, including regulation of gene transcription or inhibition by another substance. When such regulation of the non-feedback inhibited isozyme can be circumvented, however, product production is enhanced. The pathway steps selected for removing enzyme inhibition and regulation of gene expression should be limiting steps so that successful derepression will show a gain in product yield.

The invention is illustrated herein by vectors useful for producing L-phenylalanine. The specific illustrative isozymes are DAHP synthases coded by the aro genes. DAHP synthase catalyzes a chemical reaction common to biosynthesis of all three of the aromatic amino acids, phenylalanine, tyrosine and tryptophan. The aroG gene codes for a DAHP synthase that is feedback inhibited by L-phenylalanine. Other isozymes coded by aroF and aroH respectively are far less, if at all, feedback sensitive to L-phenylalanine. Accordingly, a bottleneck in the production of L-phenylalanine can be avoided by providing aroF or aroH gene product in quantities greater than would otherwise occur, and particularly in a system characterized by the absence of substances that inhibit the aroF or aroH gene products. Specifically, the expression vectors include the aroF gene, or a variant derived from it, as described in more detail below. If aroF is used to produce phenylalanine as described below, it is useful to avoid the presence of tyrosine, which inhibits the aroF gene product during the production phase, using a tyr- host. Tyr- E. coli are well-known in the art (Pittard and Wallace, (1966) J. Bacter. 9L: 1494-1508).

Where the step in the synthesis pathway at issue is also regulated through DNA sequences that control the level of expression of the gene encoding the enzyme, the vectors also embody the avoidance of such regulation. One method of avoiding regulation of the expression of the enzyme gene is to replace such regulatory DNA sequences with heterologous regulatory sequences using recombinant DNA techniques, thus circumventing the normal regulation of the biosynthetic pathway and augmenting the production of the desired biochemical beyond the point where normal regulation would have halted its further production. In order to deactivate all of the possible mechanisms for controlling gene expression, it is preferable to remove the regulatory DNA that naturally occurs with the structural gene whose expression is being derepressed and to add a foreign promoter (i.e. one that in naturally occurring structures does not govern expression of the gene).

In the preferred embodiments this can be accomplished by including the gene coding for the de-inhibited enzyme in a synthetic operon, in which the promoter is foreign to all of the operon's structural genes, or it is foreign at least to the gene selected for derepression. The two synthetic operon genes should be close to each other, but there may be short, or even quite long, sequences between them, as long as there are no signals to stop transcription between the genes. Each synthetic operon gene corresponds to the structural component of a naturally occurring gene. By the term "corresponds to," we mean that the operon gene is either identical or sufficiently related to the naturally occurring structural gene that the operon gene codes for an enzyme that catalyzes the reaction that is catalyzed by the natural gene product.

Choice of the other operon component offers an opportunity to engineer a construction that highly expresses another pathway enzyme. One preferred choice is another rate-critical pathway step. The other operon component also may be selected to simplify the cloning procedure by selecting a gene with regulatory material that is used on the vector. In that case, the 3' segment of the operon may be the derepressed structural gene, and the 5' segment may be the other member of the operon with its naturally occurring regulatory sequence.

If a promoter is used that is exogenous or foreign to both members of the operon, the members can be linked in either order. Additional structural genes may be added to the operon, and more than one of them may be feedback repressed in its naturally occurring structure, so that it also is derepressed by insertion in the operon.

The following two examples of preferred vectors pKB712 and pKB750, are illustrative.

Each vector is useful in synthesizing the amino acid L-phenylalanine not only because it contains the aroF gene in a form that is not subject to feedback transcriptional control, but also because it permits derepressed expression of the pheA gene of E. coli, which is naturally regulated in response to available phenylalanine. The product of pheA, the dual-function enzyme chorismate mutase-prephenate dehydratase, effects the penultimate steps in phenylalanine biosynthesis. Regulation of pheA expression occurs both by a repressor/operator system and by a leader peptide/attenuator system. When sufficient or excess phenylalanine is available, expression of pheA is turned off, and phenylalanine synthesis stops.

The DNA for the pheA-associated promoter/operator and leader peptide/attenuator is replaced with DNA from another promoter. Cells harboring such a construct do not halt expression of pheA nor stop phenylalanine biosynthesis in response to elevated phenylalanine. In consequence, such cells provide sufficient levels of phenylalanine to allow its secretion into the surrounding medium.

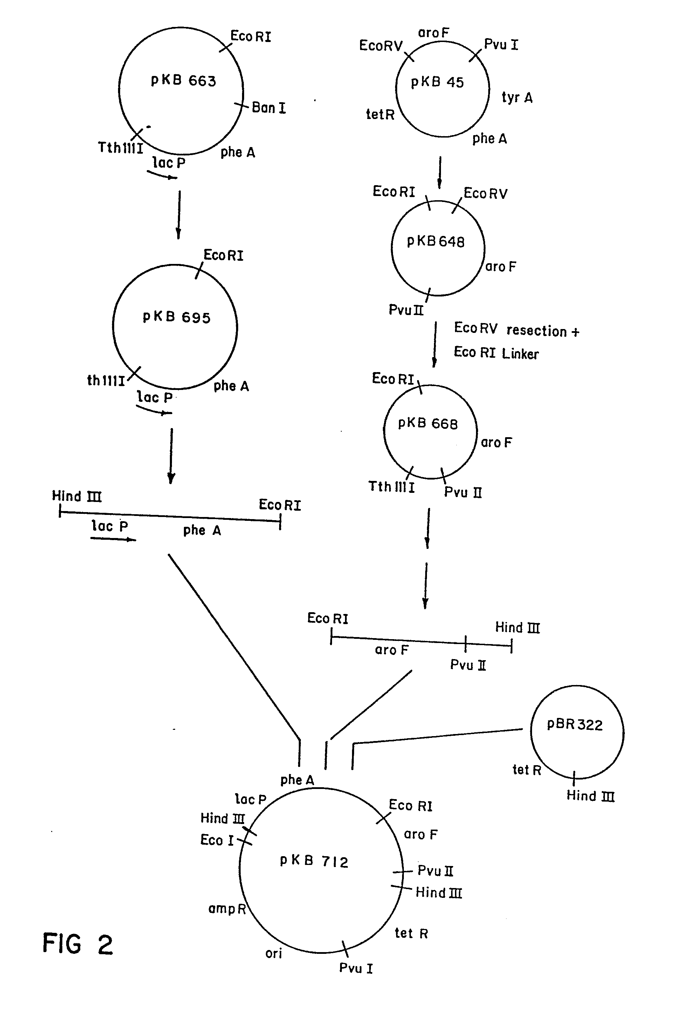

Construction of pKB712. Fig. 2 depicts the construction of pKB712 from two components:

The first component is the lac operon-pheA fusion described in the above cross-referenced parent applications with respect to construction of pKB663, as shown in Fig. 1. Plasmid pKB663 is available from strain YMC9/pKB663, which has been deposited with the American Type Culture Collection and has an accession number ATCC 39462. The BanI site just past the end of the pheA gene is converted to an EcoRI site by abutting to a filled in EcoRI site. Specifically, the resulting plasmid is treated by inserting a HindIII linker at Tth111I site 5' to the lac promoter.

The second component is the aroF structural gene selected for the reasons described above. Specifically aroF, contained on pKB45 is subcloned into pBR322 as an EcoRV to PvuII fragment, yielding pKB648. The aroF gene is preceded (5') by a EcoRV site which is cleaved and exonucleolytically resected, followed by insertion of an EcoRI linker positioned about 20-bp upstream from the aroF gene's ribosome binding site.  Near the 3' end of aroF is a PvuII site which can be joined to the PvuII site of pBR322.  The Tth111I site 3' to the gene thus cloned is changed to a HindIII by a linker.

The above two components are joined at their EcoRI ends and cloned in the HindIII site of pBR322.

In pKB712, the two structural genes are joined at a point approximately eight nucleotides after the first stop codon at the end of pheA and approximately 20 nucleotides upstream from the ribosome binding site of the aroF gene.

Construction of pKB750.  pKB750 differs from the above-described expression vector in that it is expressed under the control of the aroF promoter, not the lac promoter.

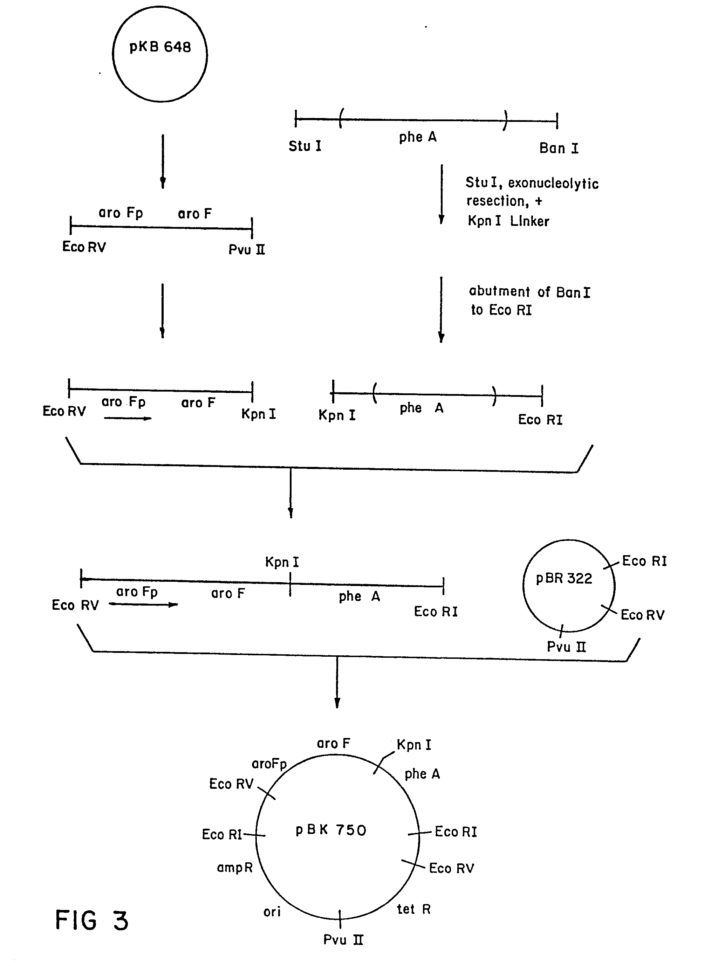

pKB750 is constructed as shown in Fig. 3 from two fragments.

The first fragment is an aroF gene with its naturally associated promoter, obtained by from pKB45 via pKB648; the segment begins with an EcoRV site 5' to the gene and ends with a KpnI site positioned next to the PvuII site at the end of aroF.

The second fragment is a pheA gene bounded by a KpnI site 5' to the gene and an EcoRI site 3' to the gene.  The KpnI site is created by cleavage at the StuI side 5' to pheA (e.g. on pKB45 or a suitable derivative) and exonucleolytic resection towards pheA; KpnI linkers are then added.  The EcoRI site is created by abutting a BanI site to a filled in EcoRI site.

These fragments are joined at their KpnI sites and cloned into pBR322 to yield pKB750.

Construction of KB358. The aroF gene product is essentially uninhibited by phenylalanine at concentrations that substantially inhibit the aroG gene product. Nevertheless, at higher phenylalanine concentrations realized using the above-described genetic constructions, the aroF gene product may lose some of its catalytic activity.

So that the DAHP-synthase-catalyzed reaction will not be a limiting factor in synthesizing phenylalanine at these higher concentrations, it is useful to obtain mutants of aroF that are not inhibited even at such higher concentrations. Using an organism that is substantially dependent on one of the aro isozymes, aroF, for synthesis of all three aromatic amino acids, it is possible to perform selection techniques described below that do not involve lethal analogue techniques (for example as described by Tsuchida in U.S Patent 4407952).

The parent strain to be used in this selection process should rely principally on the aroF gene product for synthesis of all three aromatic amino acids. This is accomplished by using any of a number of E. coli strains such as KB258, that lack the aroG gene. The aroH gene may be present because it does not direct the synthesis of sufficient DAHP synthetase to permit cell growth. If tyrosine is added to the medium at low levels (about 20 mg/liter) or if phenylalanine is added to the

It is also possible to transfer genetic material including aroF from KB358 to other strains of E. coli using known techniques and thereby to obtain the advantages of aroF in those recipient strains.

Production of Phenylalanine

Any number of strains may be used as production organisms for fermentation of phenylalanine.

E. coli K12 strain YMC9, which has been deposited with the American Type Culture Collection and has an accession number ATCC 33927 (Backman et al., Proceedings of the National Academy of Sciences USA 78 [1981], 3743-3747), is transformed with pKB712 or pKB750, and the resulting bacterium is cultured at 37°C in M9 salts plus 4 mg/ml glucose and 1 µg/ml thiamine. Incubation is continued for 10 to 15 hours, at which point phenylalanine content in the supernatant is determined.

Plasmid pKB750 or pKB712 may be used to transform strains of E. coli such as KB285 and KB280 which are described in our co-pending European Patent Application 84306842.0 (Publication No: 0140606) the disclosure of which is hereby incorporated by reference. Alternatively, pKB663 may be used to transform E. coli strains such as KB358. KB280 and KB285 are deposited with the American Type Culture Collection with accession numbers ATCC 39461 and ATCC 39463, respectively. The resulting bacterium may be used to synthesize L-phenylalanine as described in that application.

Phenylalanine concentration is determined microbiologically using phenylalanine assay medium (Difco) and Pediococcus acidilactici ATCC 8042 (Difco Manual, 1953).

Vectors pKB663, pKB712, pKB766, and strain KB358 have been deposited with the American Type Culture Collection in Rockville, Maryland and are designated by accession numbers 39,462, 39,856, 39,857, 39,858, and 53,046, respectively.

Vector pBR322 is believed sufficiently standard not to require further identification or deposit by us. Its sequence is given in Maniatis, Molecular Cloning, Cold Spring Harbor 1982. Plasmid pKB648 has not been deposited by us since any important information on it is available from pKB712.

Other embodiments are within the following claims. For example, derivatives of vectors pKB663, pKB712, and pKB750 may be used as expression vectors; or derivatives of pKB766 may be used as a precursor to an expression vector into which a suitable foreign promoter is inserted. By the term derivative we mean naturally occurring or engineered variations of the vector that conserve the desired functions.

The selection system described above and the reliance on non-feedback regulated isozymes can be applied to other biosynthetic pathways, for example, the pathways for synthesizing lysine, methionine, and threonine. Other hosts may be used for the above-described vectors.

CLAIMS

1.    A method of altering a cell to increase its production of a desired compound via a biosynthetic pathway by circumventing feedback inhibition of a first isozyme, said first isozyme catalyzing a reaction in said pathway, the catalytic activity of said first isozyme being substantially inhibited at concentrations of an inhibitor exceeding a first concentration level, said inhibitor being said desired compound or a metabolic precursor to it; said method comprising:  transforming said cell with DNA comprising a gene coding for a second isozyme, the catalytic activity of said second isozyme being substantially uninhibited at said first concentration level of said inhibitor, and said gene being transcribed at said inhibitor concentrations in amounts sufficient to enhance said product production.

2.    A method according to Claim 1, wherein said cell is a member of the species E. coli.

3.    A method according to Claim 1 or Claim 2, wherein said inhibitor is said desired compound.

4.    A method according to any preceding claim, wherein said desired compound is an aromatic amino acid.

5.    A method according to Claim 4, wherein said first isozyme and said second isozyme are each selected from the products of aroF, aroG, and aroH, said first isozyme being the product of a different gene than said second isozyme.

6.    A method according to any of Claims 1 to 3, wherein said desired compound is phenylalanine.

7.    A method according to Claim 5, wherein said first isozyme is the product of aroG and said second isozyme is the product of aroF or aroH.

8.    A method according to Claim 7 wherein said second isozyme is the product of aroF.

9.    A method according to any preceding claim, wherein transcription of said gene is feedback regulated by a transcription preventing effector, and said transcription regulation is evaded by providing said cell sufficient copies of said gene to outnumber said effector.

10. A method according to any of Claims 1 to 3, wherein said desired compound is selected from the amino acids threonine, methionine, and lysine, and said isozymes catalyze a reaction common to the biosynthetic pathways of at least two of said amino acids.

11. A cell produced by a method according to any preceding claim.

12. A cell capable of biosynthesis of a desired compound, said biosynthesis comprising a reaction catalyzed by an isozyme that is resistant to feedback inhibition by said desired compound or its metabolic precursors, said isozyme being produced in said cell by a gene whose transcription is not feedback repressed by said desired compound or its metabolic precursors, said cell having been formed by transforming a precursor cell with DNA comprising said gene.

13. A cell according to Claim 12, wherein said isozyme is the product of an aro gene and said desired compound is an aromatic amino acid.

14. A cell according to Claim 13, wherein said isozyme is the product of aroF or aroH and said desired compound is phenylalanine.

15. A method of producing a desired compound comprising culturing a cell according to Claim 11 or Claim 12 in a liquid medium and recovering said desired compound from said medium.

16. A method of producing a desired compound comprising culturing a cell according to Claim 13 or Claim 14 in a liquid medium and recovering said desired compound from said medium.

17. A method of selecting a mutant cell that exhibits increased production of a desired compound over production levels of a parent cell from which said mutant is formed, said parent cells comprising a gene coding for a feedback inhibited enzyme that catalyzes a chemical reaction common to the biosynthesis of said desired compound and of a second compound, said second compound being essential for cell growth; said method comprising: culturing said parent cell in the presence of an enzyme-inhibiting concentration of said inhibitor; and selecting mutants capable of growth under conditions such that a growth-sustaining level of said second compound is available as a result of the catalytic activity of said enzyme.

18. A method according to Claim 17, wherein said enzyme is inhibited by said desired compound.

19. A method according to Claim 17, wherein said enzyme is an isozyme that is inhibited at concentrations of an inhibitor above a level, and said parent cell lacks the ability to produce growth-sustaining amounts of said second compound via other isozymes in the presence of said inhibitor concentration level.

20. A method according to Claim 17, wherein said enzyme is DAHP synthase and said desired compound is an aromatic amino acid.

21. A method according to Claim 20, wherein said desired compound is phenylalanine and said enzyme is coded by aroF.

22. A method according to Claim 17, wherein said enzyme is an isozyme catalyzing a reaction common to the biosynthesis of at least two of the amino acids threonine, methionine, and lysine, and said desired compound is one of said amino acids.

23. A method according to Claim 17, wherein said enzyme is also feedback inhibited by said second compound and said selected mutant produces an enzyme that is not feedback inhibited either by said inhibitor or by said second compound.

24. A mutant cell produced by a method according to any of Claims 17 to 23.

25. The cell KB358 (ATCC 53,046) or a derivative thereof.

26. A method of producing a desired compound comprising: culturing a cell according to Claim 24 or a derivative of a cell according to Claim 24 in a medium, and recovering said desired compound from said medium.

0176349

# FIG. I

FIG 2

0176349

FIG 3

FIG 4